(19) Europäisches Patentamt
European Patent Office
Office européen des brevets

(11) **EP 4 091 737 A1**

(12) **EUROPÄISCHE PATENTANMELDUNG**

(43) Veröffentlichungstag:
**23.11.2022 Patentblatt 2022/47**

(21) Anmeldenummer: **22167105.0**

(22) Anmeldetag: **07.04.2022**

(51) Internationale Patentklassifikation (IPC):
**B22D 2/00** *(2006.01)* **B22D 11/18** *(2006.01)*
**B22D 11/20** *(2006.01)* **B22D 46/00** *(2006.01)*
**G01K 1/00** *(2006.01)*

(52) Gemeinsame Patentklassifikation (CPC):
**B22D 11/18; B22D 11/20; B22D 46/00; G01K 1/00**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**KH MA MD TN**

(30) Priorität: **17.05.2021 EP 21174052**
**19.11.2021 EP 21209292**

(71) Anmelder: **Primetals Technologies Austria GmbH
4031 Linz (AT)**

(72) Erfinder: **Winkler-Ebner, Bernhard
4020 Linz (AT)**

(74) Vertreter: **Metals@Linz
Primetals Technologies Austria GmbH
Intellectual Property Upstream IP UP
Turmstraße 44
4031 Linz (AT)**

(54) **UMRECHNUNG VON GEMESSENEN TEMPERATURWERTEN EINER STRANGGIESSKOKILLE**

(57) In einen von mindestens einer Kokillenwand (2) begrenzten Kokillenhohlraum (1) einer Stranggießkokille wird von oben flüssiges Metall (3) gegossen, das in dem Kokillenhohlraum (1) einen Gießspiegel (15) ausbildet. Unten wird mit einer Gießgeschwindigkeit (v) aus dem Kokillenhohlraum (1) ein zumindest teilweise erstarrter Metallstrang (4) abgezogen. In der Kokillenwand (2) sind in Einbauabständen (d) von einer dem gegossenen Metallstrang (4) zugewandten Heißseite (7) der Kokillenwand (4) Temperatursensoren (10) angeordnet, die gemessene Temperaturwerte (T) liefern. Die gemessenen Temperaturwerte (T) werden einer Auswertungseinrichtung (12) zugeführt, welche die gemessenen Temperaturwerte (T) gemäß der Beziehung $T' = T + k \cdot (d'-d)$ in errechnete Temperaturwerte (T') umrechnet. Hierbei sind T' die errechneten Temperaturwerte (T'), T die gemessenen Temperaturwerte (T), k Temperaturgradienten (k) in Dickenrichtung, d' ein einheitlicher fiktiver Abstand (d') von der Heißseite (7) der Kokillenwand (2) und d die Einbauabstände (d). Die Temperaturgradienten (k) werden von der Auswertungseinrichtung (12) in Abhängigkeit von der Gießgeschwindigkeit (v) und/oder dem Gießspiegel (15) bestimmt.

FIG 6

**Beschreibung**

Gebiet der Technik

Die vorliegende Erfindung geht aus von einem Ermittlungsverfahren für errechnete Temperaturwerte,

- wobei flüssiges Metall von oben in einen von mindestens einer Kokillenwand begrenzten Kokillenhohlraum einer Stranggießkokille gegossen wird, so dass das flüssige Metall in dem Kokillenhohlraum einen Gießspiegel ausbildet, und ein zumindest teilweise erstarrter Metallstrang mit einer Gießgeschwindigkeit unten aus dem Kokillenhohlraum abgezogen wird,
- wobei in der Kokillenwand in einem jeweiligen Einbauabstand von einer dem gegossenen Metallstrang zugewandten Heißseite der Kokillenwand ein jeweiliger Temperatursensor angeordnet ist, der einen jeweiligen gemessenen Temperaturwert liefert,
- wobei der jeweilige gemessene Temperaturwert einer Auswertungseinrichtung zugeführt wird.
Die vorliegende Erfindung geht weiterhin aus von einem Computerprogramm für eine Auswertungseinrichtung, wobei das Computerprogramm Maschinencode umfasst, der von der Auswertungseinrichtung abarbeitbar ist, wobei die Abarbeitung des Maschinencodes durch die Auswertungseinrichtung bewirkt, dass die Auswertungseinrichtung
- von Temperatursensoren, die in einer einen Kokillenhohlraum einer Stranggießkokille begrenzenden Kokillenwand in einem jeweiligen Einbauabstand von einer einem gegossenen Metallstrang zugewandten Heißseite der Kokillenwand angeordnet sind, einen jeweiligen gemessenen Temperaturwert entgegennimmt.

Die vorliegende Erfindung geht weiterhin aus von einer Auswertungseinrichtung für gemessene Temperaturwerte, wobei die Auswertungseinrichtung mit einem derartigen Computerprogramm programmiert ist.
Die vorliegende Erfindung geht weiterhin aus von einer Stranggießkokille,

- wobei die Stranggießkokille mindestens eine Kokillenwand aufweist, die einen Kokillenhohlraum begrenzt, in den von oben flüssiges Metall gegossen wird, so dass das flüssige Metall in dem Kokillenhohlraum einen Gießspiegel ausbildet, und aus dem mit einer Gießgeschwindigkeit unten ein zumindest teilweise erstarrter Metallstrang abgezogen wird,
- wobei in der Kokillenwand in einem jeweiligen Einbauabstand von einer dem gegossenen Metallstrang zugewandten Heißseite der Kokillenwand Temperatursensoren angeordnet sind, die einen jeweiligen gemessenen Temperaturwert liefern,
- wobei die Temperatursensoren zum Zuführen des jeweiligen gemessenen Temperaturwertes datentechnisch mit einer Auswertungseinrichtung der Stranggießkokille verbunden ist,
- wobei die Auswertungseinrichtung so wie obenstehend beschrieben ausgebildet ist.

Stand der Technik

Zur Überwachung des Erstarrungsprozesses beim Stranggießen sind in die Kokillenwände der Stranggießkokille Temperatursensoren eingebaut. Üblicherweise sind die Temperatursensoren in einem zweidimensionalen Raster verteilt angeordnet, wobei sich eine der beiden Dimensionen in Gießrichtung, d.h. vertikal oder im wesentlichen vertikal, erstreckt und die andere Dimension orthogonal zur Gießrichtung um den Kokillenhohlraum herum erstreckt. Die von den Temperatursensoren abgegebenen gemessenen Temperaturwerte geben Auskunft über das Temperaturgeschehen an der Heißseite der Kokillenwände. Dadurch sind Rückschlüsse auf die Schmierung des Gießprozesses und die etwaige Ausbildung von Defekten beim Stranggießen möglich. Auch Schalenhänger mit der daraus resultierenden Gefahr eines Kokillendurchbruchs sind erkennbar.
Die Temperatursensoren befinden sich in einem gewissen Abstand zur Heißseite der jeweiligen Kokillenwand, in welche sie eingebaut sind. Die gemessene Temperatur hängt stark von diesem Abstand ab. Es ist daher zur Vereinheitlichung und Vergleichbarkeit der gemessenen Temperaturwerte ein einheitlicher Abstand der Temperatursensoren von der Heißseite anzustreben.
In manchen Fällen ist ein derartiger einheitlicher Abstand ohne weiteres zu erreichen. In anderen Fällen ist es nicht möglich, einen derartigen einheitlichen Abstand zu erreichen. Beispielsweise können konstruktive Gegebenheiten wie beispielsweise die Form der Kokillenwände (Bogenkokillen oder insbesondere bei Trichterkokillen im Bereich des Trichters) und/oder Limitierungen beim Einbau der Temperatursensoren (beispielsweise notwendigerweise gerade Bohrlöcher für Glasfasersensoren) dazu führen, dass der Abstand von Temperatursensor zu Temperatursensor variiert. Auch andere Ursachen sind möglich.
Ein variierender Abstand der Temperatursensoren zur Heißseite verzerrt das von der Gesamtheit der Temperatursensoren gelieferte Temperaturbild. Dadurch wird eine Interpretation erschwert. Es kann sogar zu Fehlinterpretationen kommen. Ein weiteres Problem besteht darin, dass man eigentlich die Temperatur der Kokillenwand unmittelbar an der Heißseite bestimmen möchte. Dies ist aber technisch nicht möglich, da ein Sicherheitsabstand der Temperatursensoren zur Heißseite eingehalten werden muss. Weiterhin würden die Temperatursensoren sehr schnell zerstört werden, wenn sie Temperaturen ausgesetzt werden, wie sie in unmittelbarer Nähe der Heißseite herrschen.

Im Stand der Technik wird versucht, so weit wie möglich einen einheitlichen Abstand der Temperatursensoren von der Heißseite zu erreichen. Verbleibende Abweichungen werden im Stand der Technik oftmals hingenommen.

Aus der EP 1 103 322 A1 ist bekannt, bei einer Stranggießkokille an bestimmten Orten der Kokillenwand im gleichen Höhenbereich Thermoelemente mindestens paarweise in unterschiedlichen Tiefen der Kokillenwand anzuordnen. Aus der Temperaturdifferenz derartiger Thermoelemente wird die lokale Wärmestromdichte ermittelt. Dies erfolgt für verschiedene Höhenbereiche. Dadurch kann der Temperatur- bzw. Wärmestromverlauf über die Höhe der Kokillenwand ermittelt werden. Auch kann dadurch der maximale Temperaturverlauf an der im Kontakt mit der Schmelze befindlichen Wandfläche berechnet werden. Weiterhin kann auch die Lage des Badspiegels ermittelt werden. Entscheidend ist, dass in den Seitenwänden der Kokille in einem Bereich über und unter dem Badspiegel sowie in jeweils etwa gleichen Abständen von deren Kontaktfläche in paarweiser Anordnung Thermoelemente eingelassen sind.

Aus der DE 10 2014 227 013 A1 ist bekannt, mittels entsprechender Temperatursensoren einerseits die Temperaturen im Bereich der vertikalen Kanten der Kokillenwand und andererseits die Temperaturen im Bereich der Mitte der Kokillenwand zu erfassen. Anhand der in der Nähe der Kanten und der in der Mitte erfassten Temperaturen werden Kenngrößen ermittelt. Auf Basis der ermittelten Kenngrößen erfolgt eine Steuerung des Gießprozesses. Eine Umrechnung von erfassten Temperaturen auf einen anderen Abstand erfolgt nicht.

Aus der CN 109 365 769 A ist bekannt, in der Kokillenwand einer Stranggießkokille eine Vielzahl von Temperatursensoren zu installieren und die zugehörigen Temperaturwerte zu erfassen. Für die Temperatursensoren wird jeweils eine Temperaturcharakteristik gemäß Parametern der Gießkokille und der Einbausituation des jeweiligen Temperatursensors ermittelt. Die erfassten Temperaturwerte werden entsprechend der jeweiligen Charakteristik korrigiert. Dadurch stimmt die ermittelte Temperaturverteilung besser mit der tatsächlichen Temperaturverteilung überein. Die Korrektur dient dazu, Messfehler zu korrigieren.

Zusammenfassung der Erfindung

Die Aufgabe der vorliegenden Erfindung besteht darin, Möglichkeiten zu schaffen, mittels derer unabhängig von der Einbausituation eines jeweiligen Temperatursensors die Temperatur der Kokillenwand auf einfache Weise auf einen vorgegebenen fiktiven Abstand bezogen werden kann.

Die Aufgabe wird durch ein Ermittlungsverfahren mit den Merkmalen des Anspruchs 1 gelöst. Vorteilhafte Ausgestaltungen des Ermittlungsverfahrens sind Gegenstand der abhängigen Ansprüche 2 bis 5.

Erfindungsgemäß wird ein Ermittlungsverfahren der eingangs genannten Art dadurch ausgestaltet,

- dass die Auswertungseinrichtung für den jeweiligen Temperatursensor in Abhängigkeit von der Gießgeschwindigkeit und/oder in Abhängigkeit vom Gießspiegel einen jeweiligen Temperaturgradienten bestimmt und
- dass die Auswertungseinrichtung den jeweiligen gemessenen Temperaturwert gemäß der Beziehung

$$T' = T + k \cdot (d' - d)$$

in den jeweiligen errechneten Temperaturwert umrechnet, wobei T' der jeweilige errechnete Temperaturwert ist, T der jeweilige gemessene Temperaturwert ist, k ein jeweiliger Temperaturgradient in Dickenrichtung für den jeweiligen Temperatursensor ist, d' ein für die Temperatursensoren einheitlicher fiktiver Abstand von der Heißseite der Kokillenwand ist und d der jeweilige Einbauabstand ist.

Aufgrund der erfindungsgemäßen Vorgehensweise kann zunächst der fiktive Abstand nach Bedarf vorgegeben werden, auch als variable Vorgabe. Weiterhin kann die Auswertungseinrichtung aufgrund der Abhängigkeit des jeweiligen Temperaturgradienten von der Gießgeschwindigkeit und/oder von dem Gießspiegel den Temperaturgradienten mit hoher Genauigkeit bestimmen.

Der fiktive Abstand kann prinzipiell einen beliebigen Wert aufweisen. Vorzugsweise weist er den Wert 0 auf. Dadurch ist es möglich, die errechneten Temperaturwerte direkt für den Übergang von der Kokillenwand zur Strangschale bzw. zum noch flüssigen Metall zu ermitteln. Dadurch ist eine qualitativ besonders hochwertige Auswertung des Temperaturbildes (also der Gesamtheit der ermittelten Temperaturwerte) möglich.

In einer bevorzugten Ausgestaltung ermittelt die Auswertungseinrichtung den jeweiligen Temperaturgradienten online mittels eines Wärmeleitungsmodells der Kokillenwand, mittels dessen die Wärmeleitung in der Kokillenwand auf Basis einer Wärmeleitungsgleichung in mindestens einer Dimension modelliert wird. In diesem Fall gehen die Gießgeschwindigkeit und/oder der Gießspiegel als Eingangsgröße in das Modell ein. Bei dieser Ausgestaltung kann der Temperaturgradient nach Bedarf entsprechend der aktuellen Betriebsbedingungen der Stranggießkokille mit hoher Genauigkeit ermittelt werden. Diese Vorgehensweise führt somit zu besonders guten Ergebnissen. Erfolgt eine nur eindimensionale Modellierung, wird die Wärmeleitung nur in Dickenrichtung modelliert. Erfolgt eine zweidimensionale Modellierung, wird die Wärmeleitung auch in Höhenrichtung modelliert.

In einer einfacheren Ausgestaltung ist der jeweilige Temperaturgradient in der Auswertungseinrichtung als eindimensionale Kennlinie oder als mindestens zweidimensionales Kennlinienfeld hinterlegt. In diesem Fall bestimmt die Auswertungseinrichtung den jeweiligen Temperaturgradienten durch Auswertung der Kennlinie oder des Kennlinienfeldes. Eine eindimensionale Kennlinie kann beispielsweise bei Berücksichtigung ausschließlich der Gießgeschwindigkeit oder ausschließlich der Höhe des Gießspiegels sinnvoll sein. Ein mindestens zweidimensionales Kennlinienfeld kann beispielsweise bei Berücksichtigung sowohl der Gießgeschwindigkeit als auch der Höhe des Gießspiegels sinnvoll sein. Aufgrund der Hinterlegung der Kennlinie oder des Kennlinienfeldes ist auf einfache Weise eine Bestimmung des Temperaturgradienten möglich, ohne auf Seiten der Auswertungseinrichtung online komplexe Rechnungen durchführen zu müssen. Diese können vielmehr vorab offline durchgeführt werden.

Auch im Falle einer Ermittlung offline ist es möglich, dass der jeweilige Temperaturgradient mittels eines Wärmeleitungsmodells der Kokillenwand, mittels dessen die Wärmeleitung in der Kokillenwand auf Basis einer Wärmeleitungsgleichung in mindestens einer Dimension modelliert wird, als Funktion der Gießgeschwindigkeit und/oder als Funktion des Gießspiegels ermittelt wird. In diesem Fall werden die ermittelten Temperaturgradienten als Kennlinie oder als Kennlinienfeld in der Auswertungseinrichtung hinterlegt. Die Ermittlung kann von der Auswertungseinrichtung vorgenommen werden. Sie kann aber auch von einer anderen Einrichtung durchgeführt werden.

Die Aufgabe wird weiterhin durch ein Computerprogramm mit den Merkmalen des Anspruchs 6 gelöst. Erfindungsgemäß bewirkt die Abarbeitung des Maschinencodes, dass die Auswertungseinrichtung

- für den jeweiligen Temperatursensor in Abhängigkeit von einer Gießgeschwindigkeit, mit welcher der Metallstrang aus dem Kokillenhohlraum abgezogen wird, und/oder oder in Abhängigkeit von einem Gießspiegel, den das flüssige Metall in dem Kokillenhohlraum ausbildet, einen jeweiligen Temperaturgradienten in Dickenrichtung bestimmt und
- den jeweiligen gemessenen Temperaturwert gemäß der Beziehung

$$T' = T + k \cdot (d' - d)$$

in einen jeweiligen errechneten Temperaturwert umrechnet, wobei T' der jeweilige errechnete Temperaturwert ist, T der jeweilige gemessene Temperaturwert ist, k der jeweilige Temperaturgradient ist, d' ein für die Temperatursensoren einheitlicher fiktiver Abstand von der Heißseite der Kokillenwand ist und d der jeweilige Einbauabstand ist.

Vorzugsweise bewirkt die Abarbeitung des Maschinencodes durch die Auswertungseinrichtung sogar, dass die Auswertungseinrichtung eine der obenstehend erläuterten vorteilhaften Ausgestaltungen des Ermittlungsverfahrens realisiert.

Die Aufgabe wird weiterhin durch eine Auswertungseinrichtung mit den Merkmalen des Anspruchs 8 gelöst. Erfindungsgemäß ist die Auswertungseinrichtung mit einem erfindungsgemäßen Computerprogramm programmiert.

Die Aufgabe wird weiterhin durch eine Stranggießkokille mit den Merkmalen des Anspruchs 9 gelöst. Erfindungsgemäß ist die Auswertungseinrichtung der Stranggießkokille als erfindungsgemäße Auswertungseinrichtung ausgebildet.

Kurze Beschreibung der Zeichnungen

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die in Verbindung mit den Zeichnungen näher erläutert werden. Hierbei zeigen in schematischer Darstellung:

FIG 1     eine Stranggießkokille von der Seite,
FIG 2     die Stranggießkokille von FIG 1 von oben,
FIG 3     eine andere Stranggießkokille von oben,
FIG 4     eine Teilansicht von FIG 1,
FIG 5     einen Schnitt längs einer Linie V-V in FIG 4,
FIG 6     eine Teilansicht ähnlich der von FIG 4,
FIG 7     Temperatursensoren und eine Auswertungseinrichtung,

FIG 8      einen Schnitt längs einer Linie VIII-VIII in FIG 6,

FIG 9      eine Modifikation von FIG 8,

FIG 10      einen Schnitt längs einer Linie X-X in FIG 9,

FIG 11      eine Modifikation von FIG 7 und

FIG 12      ein Diagramm einer Wärmestromdichte,

FIG 13      ein Diagramm eines Temperaturgradienten,

FIG 14      eine Modellierung einer Wärmestromdichte und

FIG 15      eine weitere Modifikation von FIG 7.

Beschreibung der Ausführungsformen

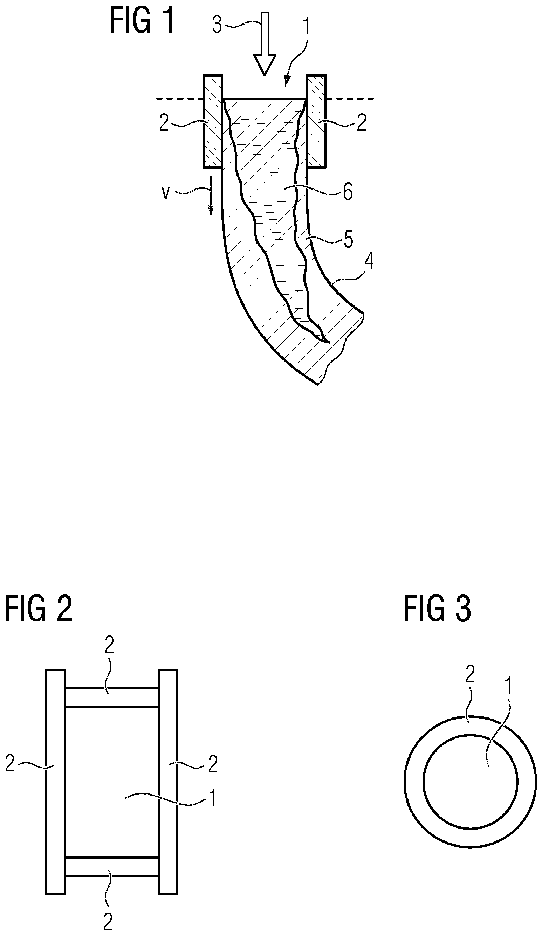

Gemäß FIG 1 wird ein Kokillenhohlraum 1 einer Stranggießkokille von mindestens einer Kokillenwand 2 begrenzt. In den Kokillenhohlraum 1 wird von oben flüssiges Metall 3 gegossen. Dadurch bildet sich in dem Kokillenhohlraum 1 ein Gießspiegel 15 aus. Das flüssige Metall 1 erstarrt an der Kokillenwand 2 (bzw. im Falle von mehreren Kokillenwänden 2 an den Kokillenwänden 2) und bildet so einen Metallstrang 4, der mit einer Gießgeschwindigkeit v nach unten aus dem Kokillenhohlraum 1 abgezogen wird. Der Metallstrang 4 weist zunächst nur eine dünne Strangschale 5 auf, die einen flüssigen Kern 6 des Metallstrangs 4 umgibt. Durch intensive Kühlung wird dem Metallstrang 4 Wärme entzogen, so dass er nach und nach durcherstarrt.

Der Gießvorgang als solcher ist so wie im Stand der Technik auch. Er muss daher nicht weiter erläutert werden. Es sei lediglich erwähnt, dass es entsprechend der Darstellung in FIG 2 möglich ist, dass mehrere Kokillenwände 2 vorhanden sind. Insbesondere bei einem Brammenquerschnitt oder einem rechteckigen Knüppelquerschnitt ist dies in der Regel der Fall. Alternativ ist es entsprechend der Darstellung in FIG 3 möglich, dass die Kokillenwand 2 die einzige Kokillenwand ist. Insbesondere bei einem runden Querschnitt des gegossenen Metallstranges 4 kann dies der Fall sein. Die Kokillenwand 2 ist in diesem Fall als Rohr ausgebildet.

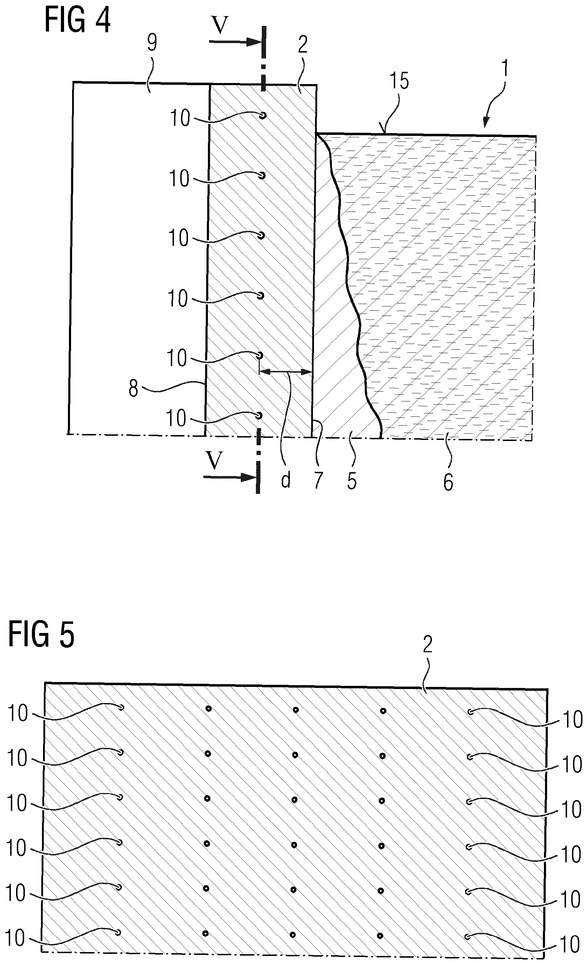

Die Kokillenwand 2 weist - siehe FIG 4 - eine Heißseite 7 und eine Kaltseite 8 auf. Die Heißseite 7 ist diejenige Seite, die dem gegossenen Metallstrang 4 zugewandt ist. Mit der Heißseite 7 grenzt die Kokillenwand 2 also an das flüssige Metall 3 oder den Metallstrang 4 bzw. die Strangschale 5 an. Die Kaltseite 8 ist die gegenüberliegende Seite der Kokillenwand 2. Im Bereich der Kaltseite 8 ist eine Wasserkühlung angeordnet (nicht dargestellt). Die Kokillenwand 2 besteht - insbesondere dann, wenn das flüssige Metall 3 Stahl ist - in der Regel aus Kupfer oder einer Kupferlegierung. An der Kaltseite 8 der Kokillenwand 2 ist oftmals eine Stützstruktur 9 angeordnet, beispielsweise eine Stahlplatte. Die Stützstruktur 9 ist im Rahmen der vorliegenden Erfindung von untergeordneter Bedeutung.

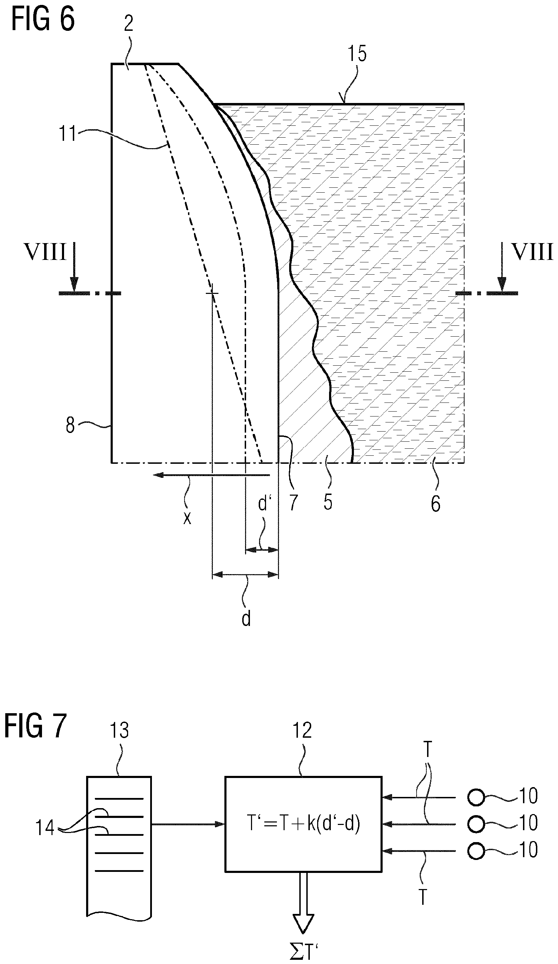

In der Regel sind entsprechend der Darstellung in den FIG 4 und 5 in der Kokillenwand 2 viele Temperatursensoren 10 angeordnet. In FIG 5 sind nur einige der Temperatursensoren 10 mit ihrem Bezugzeichen versehen, um FIG 5 nicht zu überfrachten. Oftmals sind die Temperatursensoren 10 in einem mehr oder minder regelmäßigen zweidimensionalen Raster über die Kokillenwand 2 verteilt angeordnet. Die Temperatursensoren 10 weisen - in FIG 4 nur für einen der Temperatursensoren 10 eingezeichnet - jeweils einen Abstand d von der Heißseite 7 auf. Dieser Abstand d wird nachfolgend als Einbauabstand d bezeichnet. Er kann von Temperatursensor 10 zu Temperatursensor 10 variieren. Er muss also nicht für alle Temperatursensoren 10 einheitlich derselbe sein (auch wenn dies im Einzelfall möglich ist). Beispielsweise kann entsprechend der Darstellung in FIG 6 die Kokillenwand 2 von oben nach unten gesehen an der Heißseite 7 gekrümmt sein, während die Temperatursensoren 10 von oben nach unten gesehen entlang einer geraden Linie 11 angeordnet sind.

Die Temperatursensoren 10 liefern jeweils einen gemessenen Temperaturwert T, den sie entsprechend der Darstellung in FIG 7 einer Auswertungseinrichtung 12 zuführen. Die Temperatursensoren 10 sind zu diesem Zweck datentechnisch mit der Auswertungseinrichtung 12 verbunden. Dargestellt ist dies in FIG 7 lediglich für drei der Temperatursensoren 10. In aller Regel sind aber erheblich mehr als drei Temperatursensoren 10 vorhanden.

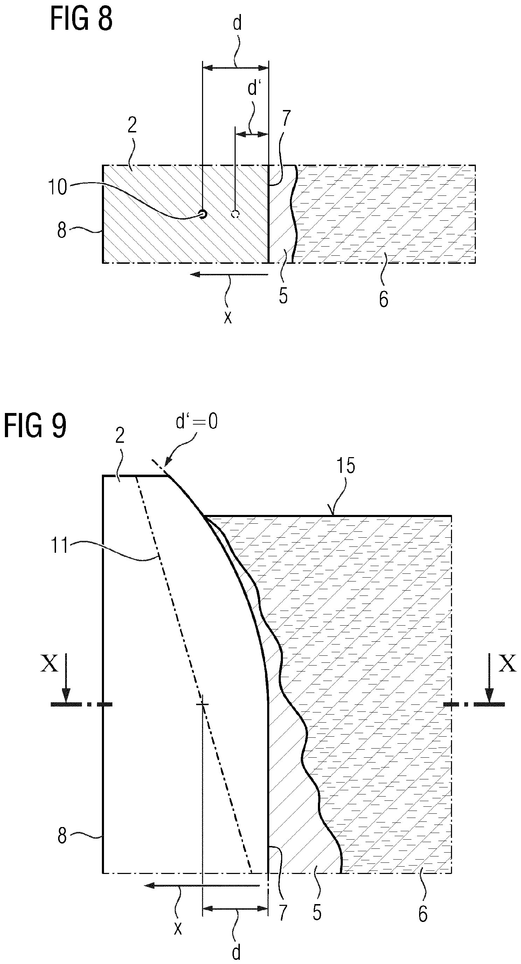

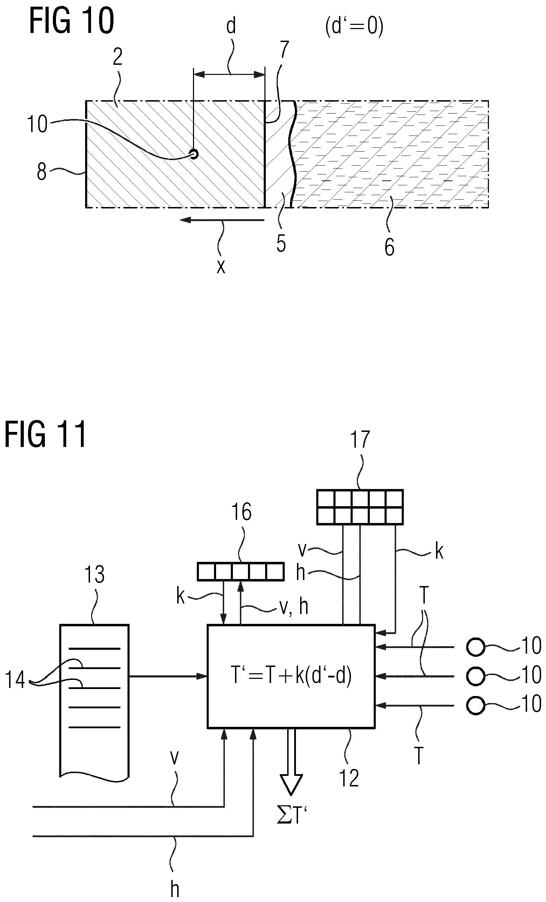

Die Auswertungseinrichtung 12 ist mit einem Computerprogramm 13 programmiert. Das Computerprogramm 13 umfasst Maschinencode 14, der von der Auswertungseinrichtung 12 abarbeitbar ist. Die Abarbeitung des Maschinencodes 14 durch die Auswertungseinrichtung 12 bewirkt, dass die Auswertungseinrichtung 12 von den Temperatursensoren 10 den jeweiligen gemessenen Temperaturwert T entgegennimmt und den jeweiligen gemessenen Temperaturwert T in einen jeweiligen errechneten Temperaturwert T' umrechnet. Die errechneten Temperaturwerte T' sind - siehe FIG 8 - auf einen fiktiven Abstand d' von der Heißseite 7 der Kokillenwand 2 bezogen. Der fiktive Abstand d' ist entsprechend der Darstellung in FIG 6 für alle Temperatursensoren 10 einheitlich derselbe. Er kann - siehe die FIG 9 und 10 - sogar den Wert 0 aufweisen, so dass die errechneten Temperaturwerte T' direkt auf die Heißseite 7 bezogen sind, also auf den Übergang von der Kokillenwand 2 zum Metallstrang 4. Die errechneten Temperaturwerte T' gibt die Auswertungseinrichtung 12 aus. Oftmals erfolgt die Ausgabe an eine Bedienperson der Stranggießanlage (nicht dargestellt). Oftmals erfolgt die Ausgabe als sogenanntes Temperaturbild, dass also die Gesamtheit der errechneten Temperaturen ausgegeben wird. Dies ist in FIG 7 durch die Angabe "ΣT" angedeutet. Das Zeichen "Σ" ist also nicht als Summe zu verstehen, sondern als Ausgeben aller oder zumindest etlicher der errechneten Temperaturwerte T'. Die Ausgabe kann beispielsweise in Form eines farbkodierten Bildes erfolgen.

Zum Bestimmen des jeweiligen errechneten Temperaturwertes T' verwertet die Auswertungseinrichtung 12 den jeweiligen gemessenen Temperaturwert T, den jeweiligen Einbauabstand d und den fiktiven Abstand d' gemäß der Beziehung

$$T' = T + k \cdot (d' - d) \tag{1}$$

k ist ein Temperaturgradient. Der Temperaturgradient k ist in Dickenrichtung der Kokillenwand 2 gerichtet und in der Regel individuell für den jeweiligen Temperatursensor 10 bestimmt.

Der Temperaturgradient k für den jeweiligen Temperatursensor 10 wird von der Auswertungseinrichtung 12 entsprechend der Darstellung in FIG 11 unter Verwertung von Betriebsparametern der Stranggießkokille bestimmt.

Beispielsweise kann der Auswertungseinrichtung 12 - siehe ergänzend FIG 4 - die Höhe h des Gießspiegels 15 bekannt sein, den das flüssige Metall 3 im Kokillenhohlraum 1 ausbildet. Beispielsweise kann der Auswertungseinrichtung 12 ein messtechnisch oder rechnerisch ermittelter Wert zugeführt werden, der angibt, wie groß der Abstand zwischen der Oberkante der Kokillenwand 2 und dem Gießspiegel 15 ist. Kurzzeitige Schwankungen der Höhe h des Gießspiegels 15 können bereits vor dem Zuführen zur Auswertungseinrichtung 12 ausgefiltert sein oder von der Auswertungseinrichtung 12 ausgefiltert werden. Wenn der Auswertungseinrichtung 12 die Höhe h des Gießspiegels 15 bekannt ist, kann die Auswertungseinrichtung 12 bei der Bestimmung des Temperaturgradienten k die Höhe h des Gießspiegels 15 berücksichtigen. Sie kann also den Temperaturgradienten k in Abhängigkeit von dem Gießspiegel 15 bestimmen.

Alternativ oder zusätzlich kann der Auswertungseinrichtung 12 die Gießgeschwindigkeit v bekannt sein. Beispielsweise kann der Auswertungseinrichtung 12 ein entsprechender messtechnisch ermittelter Wert zugeführt werden. Der Wert kann sich beispielsweise aus der Drehzahl einer Strangführungsrolle (nicht dargestellt) kurz unterhalb des Kokillenhohlraums 1 in Verbindung mit dem Durchmesser dieser Strangführungsrolle ergeben. Kurzzeitige Schwankungen der Gießgeschwindigkeit v können bereits vor dem Zuführen zur Auswertungseinrichtung 12 ausgefiltert sein oder von der Auswertungseinrichtung 12 ausgefiltert werden. Wenn der Auswertungseinrichtung 12 die Gießgeschwindigkeit v bekannt ist, kann die Auswertungseinrichtung 12 bei der Bestimmung des Temperaturgradienten k die Gießgeschwindigkeit v berücksichtigen. Sie kann also den Temperaturgradienten k in Abhängigkeit von der Gießgeschwindigkeit v bestimmen.

Zur Berücksichtigung einer funktionalen Abhängigkeit des Temperaturgradienten k von einem einzelnen Betriebsparameter der Stranggießkokille - beispielsweise entweder der Höhe h des Gießspiegels 15 oder der Gießgeschwindigkeit v - kann in der Auswertungseinrichtung 12 eine entsprechende eindimensionale Kennlinie 16 hinterlegt sein, die für bestimmte Werte des entsprechenden Betriebsparameters h, v (sogenannte Stützstellen der Kennlinie 16) den jeweiligen Wert des Temperaturgradienten k angibt. Entspricht der entsprechende Betriebsparameter h, v exakt dem Wert einer der Stützstellen, wird direkt der entsprechende Wert des Temperaturgradienten k verwendet. Liegt der entsprechende Betriebsparameter h, v zwischen zwei Stützstellen, kann der korrespondierende Wert des Temperaturgradienten k durch lineare oder nichtlineare Interpolation ermittelt werden.

Zur Berücksichtigung einer Abhängigkeit des Temperaturgradienten k von mehreren Betriebsparametern der Stranggießkokille - beispielsweise der Höhe h des Gießspiegels 15 und zusätzlich auch der Gießgeschwindigkeit v - kann in manchen Fällen in der Auswertungseinrichtung 12 ebenfalls eine entsprechende eindimensionale Kennlinie 16 hinterlegt sein, die für bestimmte Werte eines der Betriebsparameter h, v den jeweiligen Wert des Temperaturgradienten k angibt. In diesem Fall hängt der Wert, der für eine bestimmte Konfiguration von Betriebsparametern konkret ermittelt wird, funktional von dem anderen Betriebsparameter oder den anderen Betriebsparametern ab. Für die Werte des einen Betriebsparameters erfolgt wie zuvor eine Interpolation. Oftmals wird zur Berücksichtigung von mehreren Betriebsparametern der Stranggießkokille jedoch in der Auswertungseinrichtung 12 ein mindestens zweidimensionales Kennlinienfeld 17 hinterlegt sein. Die Dimensionalität des Kennlinienfeldes 17 kann insbesondere mit der Anzahl an zu berücksichtigenden Betriebsparametern übereinstimmen. Die Betriebsparameter können, wie bereits erwähnt, insbesondere die Höhe h des Gießspiegels 15 und die Gießgeschwindigkeit v sein. Bei einem mehrdimensionalen Kennlinienfeld 17 erfolgt, soweit erforderlich, eine mehrdimensionale Interpolation zwischen benachbarten Stützstellen des Stützstellenfeldes 17.

Die Anzahl an Kennlinien 16 und/oder Kennlinienfeldern 17 kann nach Bedarf bestimmt sein. Insbesondere kann für jeden Temperatursensor 10 jeweils eine eigene Kennlinie 16 bzw. ein eigenes Kennlinienfeld 17 vorhanden sein. In manchen Fällen kann die Anzahl an Kennlinien 16 bzw. Kennlinienfeldern 17 jedoch reduziert werden. Beispielsweise können zur Verringerung der Anzahl Symmetrien der Kokillenwand 2 bzw. der Kokillenwände 2 und der Anordnung der Temperatursensoren 10 ausgenutzt werden.

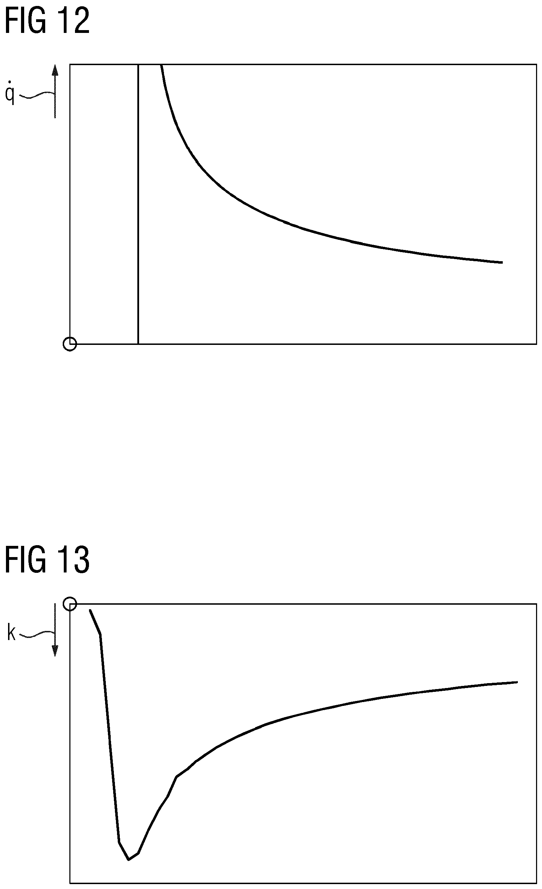

Alternativ oder zusätzlich können funktionale Beziehungen verwertet werden. Beispielsweise sind die Wärmestromdichte q und hierauf aufbauend der Temperaturgradient k oftmals in erheblichem Umfang vom Abstand δh abhängig, den ein bestimmter Temperatursensor 10 in Gießrichtung gesehen vom Gießspiegel 15 aufweist. FIG 12 zeigt zur Veranschaulichung dieses Sachverhalts den die Wärmestromdichte q als Funktion des Abstands von der Oberkante der Kokillenwand 2. Hiermit korrespondierend zeigt FIG 13 den Temperaturgradienten k als Funktion des Abstands von der Oberkante der Kokillenwand 2.

Der Temperaturgradient k für einen bestimmten Temperatursensor 10 hängt in der Regel zwar stark von der Lage des Gießspiegels 15 bzw. vom Abstand $\delta h$ des Temperatursensors 10 zum Gießspiegel 15 ab, aber nur in geringerem Umfang von der absoluten Position (in Höhenrichtung), an welcher dieser Temperatursensor 10 in der Kokillenwand 2 angeordnet ist. Aufgrund des Umstands, dass der Abstand des Gießspiegels zur Oberkante der Kokillenwand 2 variieren kann, ist die absolute Position eines Temperatursensors 10 in Höhenrichtung weniger aussagekräftig. Allerdings ändert sich der Abstand des Gießspiegels zur Kupferoberkante (Meniskus-Offset) im normalen Gießbetrieb nur um wenige Zentimeter. In vielen Fällen besteht weiterhin in Umfangsrichtung um den Kokillenhohlraum 1 herum gesehen nur eine geringe Abhängigkeit vom Ort des entsprechenden Temperatursensors 10. In derartigen Fällen kann in der Auswertungseinrichtung 12 ein einzelnes zweidimensionales Kennlinienfeld 17 hinterlegt sein, das für alle Temperatursensoren 10 gültig ist und den Wert des jeweiligen Temperatursensors 10 in Abhängigkeit von zwei Eingangsgrößen beschreibt. Die eine Eingangsgröße des Kennlinienfeldes 17 ist die Gießgeschwindigkeit v, die andere Eingangsgröße der Abstand $\delta h$. Der Abstand $\delta h$ kann für den jeweiligen Temperatursensor 10 ohne weiteres aus dessen (bekannter) absoluter Position in der Kokillenwand 2 in Verbindung mit der Höhe h des Gießspiegels 15 ermittelt werden. Falls die Abhängigkeit von der Gießgeschwindigkeit v vernachlässigt werden kann, kann weiterhin in analoger Weise für alle Temperatursensoren 10 eine eindimensionale Kennlinie 16 verwendet werden, deren Eingangsgröße der Abstand $\delta h$ ist.

Im Falle der Verwendung einer Kennlinie 16 oder eines Kennlinienfeldes 17 ist eine online-Ermittlung der Temperaturgradienten k mittels einer Modellierung der Wärmestromdichte q in der Kokillenwand 2 nicht erforderlich. Vielmehr muss zur Bestimmung des jeweiligen Temperaturgradient k von der Auswertungseinrichtung 12 für jeden Temperatursensor 10 lediglich die jeweilige Kennlinie 16 bzw. das jeweilige Kennlinienfeld 17 ausgewertet werden. Dies ist sehr schnell und mit geringem Rechenaufwand möglich.

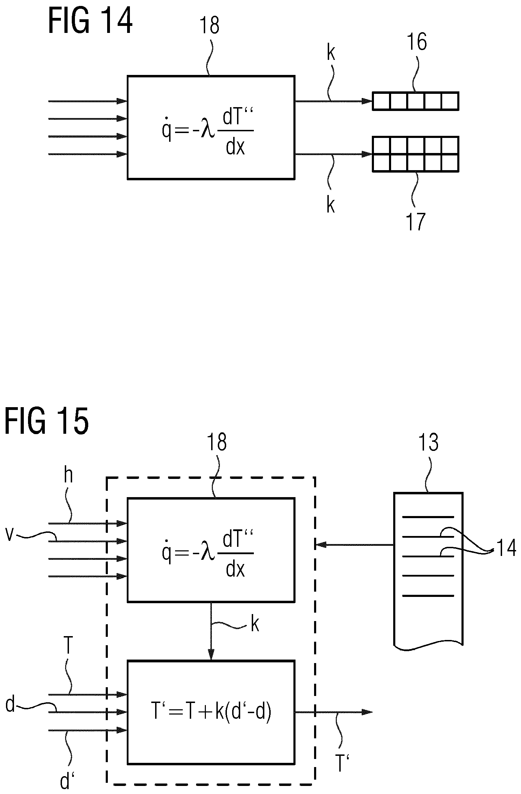

Die in den Kennlinien 16/den Kennlinienfeldern 17 hinterlegten Werte für die Temperaturgradienten k an den Stützstellen müssen selbstverständlich korrekt ermittelt werden. Dies erfolgt in der Regel entsprechend der Darstellung in FIG 14 mittels eines Wärmeleitungsmodells 18 der Kokillenwand 2, mittels dessen die Wärmeleitung in der Kokillenwand 2 auf Basis einer Wärmeleitungsgleichung modelliert wird. Die Wärmeleitung kann nach Bedarf eindimensional, zweidimensional oder dreidimensional modelliert werden. Sie erfolgt für mehrere Größen der relevanten Betriebsparameter und damit in Abhängigkeit von den relevanten Betriebsparametern. Die relevanten Betriebsparameter können, wie bereits erwähnt, insbesondere die Gießgeschwindigkeit v und die Höhe h des Gießspiegels 15 sein.

Im Falle einer eindimensionalen Modellierung kann für eine Dickenrichtung x der Kokillenwand 2 (also von der Heißseite 7 zur Kaltseite 8) eine Gleichung der Form

$$\dot{q} = -\lambda \frac{dT''}{dx} \qquad\qquad (2)$$

angesetzt werden. In Gleichung 2 sind

- q die Wärmestromdichte, die in der betrachteten Kokillenwand 2 an der betrachteten Stelle auftritt,
- A die Wärmeleitfähigkeit des Materials, aus dem die betrachtete Kokillenwand 2 besteht,
- T" die modellierte Temperatur und
- x der Ort in Dickenrichtung der Kokillenwand 2.

Wird die Wärmeleitung nur eindimensional modelliert, treten insbesondere in der Nähe des Gießspiegels 15 Fehler auf, da hier der Wärmestrom teilweise in Dickenrichtung und teilweise nach oben gerichtet ist. Eine Korrektur dieses Fehlers könnte beispielsweise derart erfolgen, dass die auf der Heißseite vorliegende Verteilung der Wärmestromdichte q korrigiert wird und mit der korrigierten Wärmestromdichte q gerechnet wird. Alternativ ist eine zwei- oder sogar dreidimensionale Modellierung möglich. Diese ist jedoch erheblich komplexer und führt oftmals nur zu unwesentlich besseren Ergebnissen. Gegebenenfalls kann es weiterhin erforderlich sein, Differenzialgleichungen anzusetzen und zu lösen.

Für das Lösen der Wärmeleitungsgleichung werden die entsprechenden Randbedingungen wie der Wärmeübergang in das Kühlwasser der Primärkühlung der Stranggießkokille und die Wärmezufuhr durch das flüssige Metall 3 benötigt. Der Wärmeübergang in das Kühlwasser der Primärkühlung korrespondiert im statischen Zustand mit der Wärmezufuhr durch das flüssige Metall 3. Er ergibt sich aus der Geometrie der Kanäle, in denen das Kühlwasser strömt, aus der Wassermenge pro Zeiteinheit, welche durch die Kanäle strömt, und weiteren Faktoren wie beispielsweise Kühlwassertemperatur.

Zum Lösen der Wärmeleitungsgleichung benötigt man weiterhin die typische Verteilung der Wärmestromdichte q über die Länge der Kokillenwand 2. Hierfür sind entsprechende Formeln und Verteilungen, die auch die Höhe h des Gießspiegels 15 berücksichtigen, aus dem Stand der Technik bekannt. Rein beispielhaft kann auf den Fachaufsatz "Heat Transfer in Funnel-mould Casting: Effect of Plate Thickness" von Begona Santillana et al., ISIJ International, Vol. 48 (2008), No. 10, Seiten 1380 bis 1388 oder das Fachbuch "Heat Withdrawal in Continuous Casting of Steel", The AISE Steel Foundation, Pittsburgh, PA, 2003 verwiesen werden. Weiterhin müssen Geometrie der Stranggießkokille und die Materialparameter (insbesondere die Wärmeleitfähigkeit und die Dichte der Kokillenwand 2 bekannt sein. Diese Größen sind jedoch unproblematisch. Mit diesen Größen kann mittels des Wärmeleitungsmodells 18 ohne weiteres die Wärmeverteilung in der jeweiligen Kokillenwand 2 ermittelt werden. Damit kann auch für jeden Ort in der Kokillenwand 2 der jeweilige Temperaturgradient k ermittelt werden.

Im Falle der Hinterlegung der Kennlinie 16 oder des Kennlinienfeldes 17 in der Auswertungseinrichtung 12 erfolgt die Modellierung offline, nach Bedarf durch die Auswertungseinrichtung 12 oder durch eine andere Einrichtung.

Es ist auch möglich, das Wärmeleitungsmodell 18 entsprechend der Darstellung in FIG 15 in die Auswertungseinrichtung 12 zu integrieren. In diesem Fall ist die Auswertungseinrichtung 12 in der Lage, für die Temperatursensoren 10 den jeweiligen Temperaturgradienten k mittels des Wärmeleitungsmodells 18 online zu ermitteln.

Die vorliegende Erfindung weist viele Vorteile auf. Zunächst ist es möglich, die Verteilung der errechneten Temperaturen T' (das sogenannte Temperaturbild) für den einheitlichen fiktiven Abstand d' von der Heißseite 7 der Kokillenwand 2 zu ermitteln. Der Einfluss von unterschiedlichen Einbauabständen d kann also kompensiert werden. Dadurch ergeben sich eine verbesserte Genauigkeit und ein konsistentes Temperaturbild. Die Gefahr von Fehlinterpretationen des Temperaturbildes wird verringert. Es ist sogar möglich, die Temperaturen T' für die Heißseite 7 selbst zu errechnen, also für die Grenzfläche von der Kokillenwand 2 zum Metallstrang 4. Dies gestattet einen optimalen Einblick in den Stranggießprozess. Der erfindungsgemäße Algorithmus ist leicht zu implementieren und kann ohne weiteres aktiviert und deaktiviert werden. Es ist sogar möglich, den fiktiven Abstand d' vorgebbar zu gestalten, dass also eine Bedienperson den fiktiven Abstand d' vorgibt.

Obwohl die Erfindung im Detail durch das bevorzugte Ausführungsbeispiel näher illustriert und beschrieben wurde, so ist die Erfindung nicht durch die offenbarten Beispiele eingeschränkt und andere Varianten können vom Fachmann hieraus abgeleitet werden, ohne den Schutzumfang der Erfindung zu verlassen.

Bezugszeichenliste

| | |
|---|---|
| 1 | Kokillenhohlraum |
| 2 | Kokillenwände |
| 3 | flüssiges Metall |
| 4 | Metallstrang |
| 5 | Strangschale |
| 6 | flüssiger Kern |
| 7 | Heißseite |
| 8 | Kaltseite |
| 9 | Stützstruktur |
| 10 | Temperatursensoren |
| 11 | gerade Linie |
| 12 | Auswertungseinrichtung |
| 13 | Computerprogramm |
| 14 | Maschinencode |
| 15 | Gießspiegel |
| 16 | Kennlinie |
| 17 | Kennlinienfeld |
| 18 | Wärmeleitungsmodell |
| d | Einbauabstände |
| d' | fiktiver Abstand |
| h | Höhe des Gießspiegels |
| k | Temperaturgradient |
| $\dot{q}$ | Wärmestromdichte |
| T | gemessene Temperaturwerte |
| T' | errechnete Temperaturwerte |
| T" | modellierte Temperatur |

v    Gießgeschwindigkeit

x    Dickenrichtung

δh    Abstand

A    Wärmeleitfähigkeit

**Patentansprüche**

1. Ermittlungsverfahren für errechnete Temperaturwerte (T'),

- wobei flüssiges Metall (3) von oben in einen von mindestens einer Kokillenwand (2) begrenzten Kokillenhohlraum (1) einer Stranggießkokille gegossen wird, so dass das flüssige Metall (3) in dem Kokillenhohlraum (1) einen Gießspiegel (15) ausbildet, und ein zumindest teilweise erstarrter Metallstrang (4) mit einer Gießgeschwindigkeit (v) unten aus dem Kokillenhohlraum (1) abgezogen wird,
- wobei in der Kokillenwand (2) in einem jeweiligen Einbauabstand (d) von einer dem gegossenen Metallstrang (4) zugewandten Heißseite (7) der Kokillenwand (4) ein jeweiliger Temperatursensor (10) angeordnet ist, der einen jeweiligen gemessenen Temperaturwert (T) liefert,
- wobei der jeweilige gemessene Temperaturwert (T) einer Auswertungseinrichtung (12) zugeführt wird,
- wobei die Auswertungseinrichtung (12) für den jeweiligen Temperatursensor (10) in Abhängigkeit von der Gießgeschwindigkeit (v) und/oder in Abhängigkeit vom Gießspiegel (15) einen jeweiligen Temperaturgradienten (k) bestimmt und
- wobei die Auswertungseinrichtung (12) den jeweiligen gemessenen Temperaturwert (T) gemäß der Beziehung

$$T' = T + k \cdot (d' - d)$$

in den jeweiligen errechneten Temperaturwert (T') umrechnet, wobei T' der jeweilige errechnete Temperaturwert (T') ist, T der jeweilige gemessene Temperaturwert (T) ist, k ein jeweiliger Temperaturgradient (k) in Dickenrichtung für den jeweiligen Temperatursensor (10) ist, d' ein für die Temperatursensoren (10) einheitlicher fiktiver Abstand (d') von der Heißseite (7) der Kokillenwand (2) ist und d der jeweilige Einbauabstand (d) ist.

2. Ermittlungsverfahren nach Anspruch 1,
**dadurch gekennzeichnet,**
**dass** der fiktive Abstand (d') den Wert 0 aufweist.

3. Ermittlungsverfahren nach Anspruch 1 oder 2,
**dadurch gekennzeichnet,**
**dass** die Auswertungseinrichtung (12) den jeweiligen Temperaturgradienten (k) mittels eines Wärmeleitungsmodells (18) der Kokillenwand (2), mittels dessen die Wärmeleitung in der Kokillenwand (2) auf Basis einer Wärmeleitungsgleichung in mindestens einer Dimension modelliert wird, online ermittelt.

4. Ermittlungsverfahren nach Anspruch 1 oder 2,
**dadurch gekennzeichnet,**
**dass** der jeweilige Temperaturgradient (k) in der Auswertungseinrichtung (12) als eindimensionale Kennlinie (16) oder als mindestens zweidimensionales Kennlinienfeld (17) hinterlegt ist und dass die Auswertungseinrichtung (12) den jeweiligen Temperaturgradienten (k) durch Auswertung der Kennlinie (16) oder des Kennlinienfeldes (17) bestimmt.

5. Ermittlungsverfahren nach Anspruch 4,
**dadurch gekennzeichnet,**
**dass** der jeweilige Temperaturgradient (k) mittels eines Wärmeleitungsmodells (18) der Kokillenwand (2), mittels dessen die Wärmeleitung in der Kokillenwand (2) auf Basis einer Wärmeleitungsgleichung in mindestens einer Dimension modelliert wird, als Funktion der Gießgeschwindigkeit (v) und/oder als Funktion des Gießspiegels (15) offline ermittelt wird und als Kennlinie (16) oder als Kennlinienfeld (17) in der Auswertungseinrichtung (12) hinterlegt wird.

6. Computerprogramm für eine Auswertungseinrichtung (12), wobei das Computerprogramm Maschinencode (14) umfasst, der von der Auswertungseinrichtung (12) abarbeitbar ist, wobei die Abarbeitung des Maschinencodes (14) durch die Auswertungseinrichtung (12) bewirkt, dass die Auswertungseinrichtung (12)

- von Temperatursensoren (10), die in einer einen Kokillenhohlraum (1) einer Stranggießkokille begrenzenden Kokillenwand (2) in einem jeweiligen Einbauabstand (d) von einer einem gegossenen Metallstrang (4) zugewandten Heißseite (7) der Kokillenwand (2) angeordnet sind, einen jeweiligen gemessenen Temperaturwert (T) entgegennimmt,
- für den jeweiligen Temperatursensor (10) in Abhängigkeit von einer Gießgeschwindigkeit (v), mit welcher der Metallstrang (4) aus dem Kokillenhohlraum (1) abgezogen wird, und/oder in Abhängigkeit von einem Gießspiegel (15), den das flüssige Metall (3) in dem Kokillenhohlraum (1) ausbildet, einen jeweiligen Temperaturgradienten (k) in Dickenrichtung bestimmt und
- den jeweiligen gemessenen Temperaturwert (T) gemäß der Beziehung

$$T' = T + k \cdot (d' - d)$$

in einen jeweiligen errechneten Temperaturwert (T') umrechnet, wobei T' der jeweilige errechnete Temperaturwert (T') ist, T der jeweilige gemessene Temperaturwert (T) ist, k der jeweilige Temperaturgradient (k) ist, d' ein für die Temperatursensoren (10) einheitlicher fiktiver Abstand (d') von der Heißseite (7) der Kokillenwand (2) ist und d der jeweilige Einbauabstand (d) ist.

7. Computerprogramm nach Anspruch 6,
**dadurch gekennzeichnet,**
**dass** die Abarbeitung des Maschinencodes (14) durch die Auswertungseinrichtung (12) bewirkt, dass die Auswertungseinrichtung (12) die zusätzlichen Maßnahmen mindestens eines der Ansprüche 2 bis 5 ausführt.

8. Auswertungseinrichtung für gemessene Temperaturwerte (T), wobei die Auswertungseinrichtung mit einem Computerprogramm nach Anspruch 6 oder 7 programmiert ist.

9. Stranggießkokille,

- wobei die Stranggießkokille mindestens eine Kokillenwand (2) aufweist, die einen Kokillenhohlraum (1) begrenzt, in den von oben flüssiges Metall (3) gegossen wird, so dass das flüssige Metall (3) in dem Kokillenhohlraum (1) einen Gießspiegel (15) ausbildet, und aus dem mit einer Gießgeschwindigkeit (v) unten ein zumindest teilweise erstarrter Metallstrang (4) abgezogen wird,
- wobei in der Kokillenwand (2) in einem jeweiligen Einbauabstand (d) von einer dem gegossenen Metallstrang (4) zugewandten Heißseite (7) der Kokillenwand (2) Temperatursensoren (10) angeordnet sind, die einen jeweiligen gemessenen Temperaturwert (T) liefern,
- wobei die Temperatursensoren (10) zum Zuführen des jeweiligen gemessenen Temperaturwertes (T) datentechnisch mit einer Auswertungseinrichtung (12) der Stranggießkokille verbunden ist,
- wobei die Auswertungseinrichtung (12) gemäß Anspruch 8 ausgebildet ist.

# FIG 1

# FIG 2

# FIG 3

FIG 4

FIG 5

FIG 6

VIII

VIII

d'

d

x

T'=T+k(d'-d)

ΣT'

FIG 7

T

T

10

10

10

FIG 8

FIG 9

## FIG 10

(d'=0)

d

2

7

10

8

5

X

6

## FIG 11

17

16

13

14

v
h

k

k

v, h

v

k

T

T'=T+k(d'-d)

10

10

10

12

ΣT'

v

h

T

FIG 12

$\dot{q}$

FIG 13

$k$

FIG 14

18

$$\dot{q} = -\lambda \frac{dT''}{dx}$$

k

16

k

17

FIG 15

18

h

v

$$\dot{q} = -\lambda \frac{dT''}{dx}$$

13

14

k

T

d

$$T' = T + k(d'-d)$$

d'

T'

Europäisches
Patentamt

European
Patent Office

Office européen
des brevets

## EUROPÄISCHER RECHERCHENBERICHT

**Nummer der Anmeldung**

**EP 22 16 7105**

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 1 103 322 A1 (SMS DEMAG AG [DE]) 30. Mai 2001 (2001-05-30) * Abbildung 1 * * Absatz [0001] – Absatz [0004] * * Absatz [0009] – Absatz [0019] * * Ansprüche 1-7 * ----- | 1-9 | INV. B22D2/00 B22D11/18 B22D11/20 B22D46/00 G01K1/00 |
| X | DE 10 2014 227013 A1 (SMS GROUP GMBH [DE]) 24. März 2016 (2016-03-24) * Abbildungen 1-5 * * Absätze [0001] – [0005] * * Absatz [0011] – Absatz [0023] * * Absatz [0031] – Absatz [0052] * ----- | 1-9 | |
| X | CN 109 365 769 A (UNIV CHONGQING POSTS & TELECOM) 22. Februar 2019 (2019-02-22) * Abbildungen 1-5 * * Absatz [0001] * * Absatz [0009] – Absatz [0037] * * Absatz [0043] – Absatz [0072] * ----- | 1-9 | |

**RECHERCHIERTE SACHGEBIETE (IPC)**

B22D
G01W
G01K

Der vorliegende Recherchenbericht wurde für alle Patentansprüche erstellt

| Recherchenort | Abschlußdatum der Recherche | Prüfer |
|---|---|---|
| Den Haag | 3. Oktober 2022 | Jung, Régis |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

........................................................................

& : Mitglied der gleichen Patentfamilie, übereinstimmendes Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 22 16 7105

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

03-10-2022

| Im Recherchenbericht angeführtes Patentdokument | Datum der Veröffentlichung | Mitglied(er) der Patentfamilie | Datum der Veröffentlichung |
|---|---|---|---|
| EP 1103322 A1 | 30-05-2001 | AT 291980 T | 15-04-2005 |
| | | DE 19956577 A1 | 31-05-2001 |
| | | EP 1103322 A1 | 30-05-2001 |
| | | JP 2001334354 A | 04-12-2001 |
| | | US 6776217 B1 | 17-08-2004 |
| DE 102014227013 A1 | 24-03-2016 | KEINE | |
| CN 109365769 A | 22-02-2019 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1103322 A1
- DE 102014227013 A1
- CN 109365769 A

**In der Beschreibung aufgeführte Nicht-Patentliteratur**

- **BEGONA SANTILLANA et al.** Heat Transfer in Funnel-mould Casting: Effect of Plate Thickness. *ISIJ International,* 2008, vol. 48 (10), 1380-1388
- Heat Withdrawal in Continuous Casting of Steel. Fachbuch. The AISE Steel Foundation, 2003